Europäisches Patentamt

European Patent Office

Office européen des brevets

(19)

(11) **EP 1 213 357 A2**

(12) **EUROPEAN PATENT APPLICATION**

(43) Date of publication:
**12.06.2002 Bulletin 2002/24**

(51) Int Cl.⁷: **C12Q 1/26**, C12Q 1/48, C12Q 1/34

(21) Application number: **01308658.2**

(22) Date of filing: **10.10.2001**

| (84) Designated Contracting States:<br>**AT BE CH CY DE DK ES FI FR GB GR IE IT LI LU MC NL PT SE TR**<br>Designated Extension States:<br>**AL LT LV MK RO SI**<br><br>(30) Priority: **11.10.2000 JP 2000310685**<br><br>(71) Applicant: **KYOWA MEDEX CO., LTD.**<br>**Tokyo 104-0042 (JP)** | (72) Inventors:<br>• **Sasaki, Mihoko, c/o Kyowa Medex Research Lab.**<br>  **Sunto-gun, Shizuoka 411-0932 (JP)**<br>• **Tazoe, Sakae, c/o Tokyo Research Laboratories**<br>  **Machida-shi, Tokyo 194-8533 (JP)**<br>• **Miike, Akira, c/o Kyowa Medex Research Lab.**<br>  **Sunto-gun, Shizuoka 411-0932 (JP)**<br><br>(74) Representative: **Tanner, James Percival**<br>**D. Young & Co,**<br>**21 New Fetter Lane**<br>**London EC4A 1DA (GB)** |

(54) **Method and reagent for quantitative determination of 1, 5-Anhydroglucitol**

(57) The present invention provides a method for determining 1,5-anhydroglucitol (1,5-AG) in a sample containing 1,5-AG, maltose and glucose, which comprises: converting maltose in the sample into glucose using an enzyme system capable of converting maltose into glucose; converting glucose into a compound which is not phosphorylated by 1,5-anhydroglucitol 6-phosphorylating enzyme system (AG-6P-ES) or dehydrogenated by the action of 1,5-anhydroglucitol-6-phosphate dehydrogenase (AG-6PDH), using an enzyme system capable of converting glucose into said compound; converting 1,5-AG into 1,5-anhydroglucitol-6-phosphate (1,5-AG-6P) using the AG-6P-ES; dehydrogenating the formed 1,5-AG-6P with AG-6PDH in the presence of an oxidized coenzyme; and determining the component formed or reduced by the dehydrogenation reaction. A reagent and a reagent kit useful in this method are also provided.

Fig. 1

**EP 1 213 357 A2**

**Description**

Background of the Invention

The present invention relates to a method for the quantitative determination of 1,5-anhydroglucitol (hereinafter abbreviated as 1,5-AG) which is useful as a diagnostic marker for diabetes, and a reagent and a reagent kit for use therein.

1,5-AG, which is present in biological samples such as human body fluids, is useful as a diagnostic marker for diabetes and bears a close similarity in structure to glucose. Glucose is usually contained in biological samples at a concentration which is over 10 times higher than that of 1,5-AG, and thus interferes with the determination of 1,5-AG using an enzyme.

Previous methods for the enzymatic determination of 1,5-AG in a sample include methods in which the presence of glucose in the sample is ignored and methods in which glucose is eliminated prior to the determination of 1,5-AG. 1,5-AG cannot be accurately determined by the former methods; in the latter methods, the enzyme used for the elimination of glucose sometimes affects the determination of 1,5-AG. EP-1008657A1 discloses a method for the determination of 1,5-AG in a sample which comprises converting glucose into fructose 1,6-diphosphate, converting 1,5-AG into 1,5-anhydroglucitol-6-phosphate by phosphorylation, dehydrogenating the formed 1,5-anhydroglucitol-6-phosphate with 1,5-anhydroglucitol-6-phosphate dehydrogenase in the presence of an oxidized coenzyme, and then determining the formed reduced coenzyme. In this method, fructose 1,6-diphosphate converted from glucose is not reconverted into glucose and has no influence on the enzyme reactions for the 1,5-AG determination. Thus, this method is an excellent method capable of accurately determining 1,5-AG in a sample.

However, this publication is silent about maltose in a sample. Infusions containing maltose are often administered to patients of certain diseases including diabetes for the purpose of energy supply, and so maltose is contained in biological samples from such patients. Further, the enzyme used for the 1,5-AG determination sometimes also possesses the activity to convert maltose into glucose, for example, α-glucosidase activity, due to incomplete purification, etc. In such cases, the presence of maltose affects the 1,5-AG determination, which necessitates effecting elimination of maltose in quantitative assay of biological samples.

Japanese Published Unexamined Patent Application No. 70795/94 discloses a method for the determination of 1,5-AG using pyranose oxidase in which maltose is eliminated, prior to the 1,5-AG determination, by converting maltose into glucose using α-glucosidase and then eliminating glucose using hexokinase and ATP under specific conditions. However, this method requires the selective elimination of glucose so that 1,5-AG may remain and is defective in that the operations are complicated and the results are not highly accurate.

Japanese Published Unexamined Patent Application No. 7197/94 discloses a method for the determination of 1,5-AG in which maltose in a sample is converted into glucose using α-glucosidase, glucose is converted into glucose 6-phosphate using glucokinase or hexokinase, and then 1,5-AG is determined using pyranose oxidase and L-sorbose oxidase. In the phosphorylation of glucose, 1,5-AG is simultaneously phosphorylated by glucokinase or hexokinase, and therefore, accurate determination of 1,5-AG cannot be expected.

Summary of the Invention

According to the present invention, 1,5-AG in a sample can be accurately determined by converting maltose in the sample into glucose (conversion of maltose into glucose), converting glucose into a compound which is not affected by the enzymes used in the 1,5-AG determination (elimination of glucose), converting 1,5-AG into 1,5-anhydroglucitol-6-phosphate (hereinafter referred to as 1,5-AG-6P) (phosphorylation of 1,5-AG), dehydrogenating 1,5-AG-6P with 1,5-anhydroglucitol-6-phosphate dehydrogenase in the presence of an oxidized coenzyme (dehydrogenation of 1,5-AG-6P), and then determining the component formed or reduced by the dehydrogenation reaction.

The present invention relates to the following (1) to (34).

(1) A method for determining 1,5-AG in a sample containing 1,5-AG, maltose and glucose, which comprises:

converting maltose in the sample into glucose using an enzyme system capable of converting maltose into glucose [hereinafter referred to as enzyme system (A)];
converting glucose into a compound which is not phosphorylated by 1,5-anhydroglucitol 6-phosphorylating enzyme system (hereinafter referred to as AG-6P-ES) or dehydrogenated by the action of 1,5-anhydroglucitol-6-phosphate dehydrogenase (hereinafter referred to as AG-6PDH), using an enzyme system capable of converting glucose into said compound [hereinafter referred to as enzyme system (B)];
converting 1,5-AG into 1,5-AG-6P using the AG-6P-ES;
dehydrogenating the formed 1,5-AG-6P with AG-6PDH in the presence of an oxidized coenzyme; and

determining the component formed or reduced by the dehydrogenation reaction.

(2) The method according to (1), wherein the AG-6P-ES is selected from the group consisting of (a) hexokinase and NTP, (b) glucokinase and NTP, and (c) NDP-dependent hexokinase and NDP.

(3) The method according to (1), wherein enzyme system (A) is selected from the group consisting of (a) α-glucosidase, (b) maltose phosphorylase and inorganic phosphorus, and (c) maltose phosphorylase, inorganic phosphorus and maltose 1-epimerase.

(4) The method according to (1), wherein enzyme system (B) is selected from the group consisting of (a) glucose 6-phosphorylating enzyme system (hereinafter referred to as G-6P-ES), phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

(5) The method according to (1), wherein the AG-6P-ES is selected from the group consisting of (a) hexokinase and NTP, (b) glucokinase and NTP, and (c) NDP-dependent hexokinase and NDP; enzyme system (A) is selected from the group consisting of (a) α-glucosidase, (b) maltose phosphorylase and inorganic phosphorus, and (c) maltose phosphorylase, inorganic phosphorus and maltose 1-epimerase; and enzyme system (B) is selected from the group consisting of (a) G-6P-ES, phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

(6) The method according to (4) or (5), wherein the G-6P-ES is selected from the group consisting of (a) hexokinase and NTP, (b) glucokinase and NTP, and (c) NDP-dependent hexokinase and NDP.

(7) The method according to (1), wherein the AG-6PDH is derived from a microorganism belonging to the genus Escherichia.

(8) The method according to (1), wherein the formed component is a reduced coenzyme and the determination of the reduced coenzyme is carried out by measuring the absorbance of the reaction mixture obtained by dehydrogenation.

(9) The method according to (1), wherein the formed component is a reduced coenzyme and the determination of the reduced coenzyme is carried out by subjecting the reduced coenzyme to reaction with a tetrazolium salt in the presence of an electron carrier and then measuring the absorbance of the reaction mixture colored by the formed formazan pigment.

(10) A reagent for the determination of 1,5-AG, comprising:

a reagent for the conversion of maltose into glucose comprising enzyme system (A) capable of converting maltose into glucose;
a reagent for the elimination of glucose comprising enzyme system (B) capable of converting glucose into a compound which is not phosphorylated by AG-6P-ES or dehydrogenated by AG-6PDH;
a reagent for the conversion of 1,5-AG into 1,5-AG-6P comprising AG-6P-ES; and
a reagent for the dehydrogenation of 1,5-AG-6P comprising AG-6PDH and an oxidized coenzyme.

(11) The reagent according to (10), wherein the AG-6P-ES is selected from the group consisting of (a) hexokinase and NTP, (b) glucokinase and NTP, and (c) NDP-dependent hexokinase and NDP.

(12) The reagent according to (10), wherein enzyme system (A) is selected from the group consisting of (a) α-glucosidase, (b) maltose phosphorylase and inorganic phosphorus, and (c) maltose phosphorylase, inorganic phosphorus and maltose 1-epimerase.

(13) The reagent according to (10), wherein enzyme system (B) is selected from the group consisting of (a) G-6P-ES, phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

(14) The reagent according to (10), wherein the AG-6P-ES is selected from the group consisting of (a) hexokinase and NTP, (b) glucokinase and NTP, and (c) NDP-dependent hexokinase and NDP; enzyme system (A) is selected from the group consisting of (a) α-glucosidase, (b) maltose phosphorylase and inorganic phosphorus, and (c) maltose phosphorylase, inoganic phosphorus and maltose 1-epimerase; and enzyme system (B) is selected from the group consisting of (a) G-6P-ES, phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

(15) The reagent according to (13) or (14), wherein the G-6P-ES is selected from the group consisting of (a) hexokinase and NTP, (b) glucokinase and NTP, and (c) NDP-dependent hexokinase and NDP.

(16) The reagent according to (10), wherein the AG-6PDH is derived from a microorganism belonging to the genus Escherichia.

(17) A reagent kit for the determination of 1,5-AG, comprising:

a first container which contains a reagent for the conversion of maltose into glucose comprising enzyme system (A) capable of converting maltose into glucose, a reagent for the elimination of glucose comprising enzyme system (B) capable of converting glucose into a compound which is not phosphorylated by AG-6P-ES or dehydrogenated by AG-6PDH, and a reagent for the conversion of 1,5-AG into 1,5-AG-6P comprising AG-6P-ES; and
a second container which contains a reagent for the dehydrogenation of 1,5-AG-6P comprising AG-6PDH and an oxidized coenzyme.

(18) A reagent kit for the determination of 1,5-AG, comprising:

a first container which contains a reagent for the conversion of maltose into glucose comprising enzyme system (A) capable of converting maltose into glucose, and a reagent for the elimination of glucose comprising enzyme system (B) capable of converting glucose into a compound which is not phosphorylated by AG-6P-ES or dehydrogenated by AG-6PDH; and
a second container which contains a reagent for the conversion of 1,5-AG into 1,5-AG-6P comprising AG-6P-ES, and a reagent for the dehydrogenation of 1,5-AG-6P comprising AG-6PDH and an oxidized coenzyme.

(19) A reagent kit for the determination of 1,5-AG, comprising:

a first container which contains a reagent for the conversion of maltose into glucose comprising enzyme system (A) capable of converting maltose into glucose, a reagent for the elimination of glucose comprising enzyme system (B) capable of converting glucose into a compound which is not phosphorylated by AG-6P-ES or dehydrogenated by AG-6PDH, a reagent for the conversion of 1,5-AG into 1,5-AG-6P comprising AG-6P-ES, and an oxidized coenzyme; and
a second container which contains AG-6PDH.

(20) A reagent kit for the determination of 1,5-AG, comprising:

a first container which contains a reagent for the conversion of maltose into glucose comprising enzyme system (A) capable of converting maltose into glucose, a reagent for the elimination of glucose comprising enzyme system (B) capable of converting glucose into a compound which is not phosphorylated by AG-6P-ES or dehydrogenated by AG-6PDH, and an oxidized coenzyme; and
a second container which contains a reagent for the conversion of 1,5-AG into 1,5-AG-6P comprising AG-6P-ES, and AG-6PDH.

(21) A reagent kit for the determination of 1,5-AG, comprising:

a first container which contains a reagent for the conversion of maltose into glucose comprising enzyme system (A) capable of converting maltose into glucose, a reagent for the elimination of glucose and the conversion of 1,5-AG into 1,5-AG-6P comprising a member selected from the group consisting of (a) hexokinase and NTP, (b) glucokinase and NTP, and (c) NDP-dependent hexokinase and NDP, and phosphohexose isomerase, phosphofructokinase, NTP and an oxidized coenzyme; and
a second container which contains AG-6PDH.

(22) A reagent kit for the determination of 1,5-AG, comprising:

a first container which contains a reagent for the conversion of maltose into glucose comprising enzyme system (A) capable of converting maltose into glucose, a reagent for the elimination of glucose comprising enzyme system (B) selected from the group consisting of (b) glucose oxidase, (c) glucose oxidase and mutarotase, (d) glucose oxidase and catalase, and (e) glucose oxidase, mutarotase and catalase, and NTP or NDP and an oxidized coenzyme; and
a second container which contains an enzyme capable of converting 1,5-AG into 1,5-AG-6P, and AG-6PDH.

(23) The reagent kit according to any of (17) to (20), wherein the AG-6P-ES is selected from the group consisting of (a) hexokinase and NTP, (b) glucokinase and NTP, and (c) NDP-dependent hexokinase and NDP.

(24) The reagent kit according to any of (17) to (22), wherein enzyme system (A) is selected from the group consisting of (a) α-glucosidase, (b) maltose phosphorylase and inorganic phosphorus, and (c) maltose phosphorylase, inorganic phosphorus and maltose 1-epimerase.

(25) The reagent kit according to any of (17) to (20), wherein enzyme system (B) is selected from the group consisting of (a) G-6P-ES, phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

(26) The reagent kit according to any of (17) to (20), wherein the AG-6P-ES is selected from the group consisting of (a) hexokinase and NTP, (b) glucokinase and NTP, and (c) NDP-dependent hexokinase and NDP; enzyme system (A) is selected from the group consisting of (a) α-glucosidase, (b) maltose phosphorylase and inorganic phosphorus, and (c) maltose phosphorylase, inoganic phosphorus and maltose 1-epimerase; and enzyme system (B) is selected from the group consisting of (a) G-6P-ES, phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

(27) The reagent kit according to (25) or (26), wherein the G-6P-ES is selected from the group consisting of (a) hexokinase and NTP, (b) glucokinase and NTP, and (c) NDP-dependent hexokinase and NDP.

(28) The reagent kit according to any of (17) to (22), wherein the AG-6PDH is derived from a microorganism belonging to the genus Escherichia.

(29) The reagent kit according to any of (17) to (22), wherein the first container further contains an electron carrier and the second container further contains a tetrazolium salt.

(30) A method for determining 1,5-AG in a sample containing 1,5-AG, maltose and glucose, which comprises:

converting maltose in the sample into glucose with α-glucosidase in an aqueous medium;
converting glucose in the sample and glucose formed in the medium into fructose-1,6-diphosphate with ADP-dependent hexokinase, phosphohexose isomerase and 6-phosphofructokinase in the presence of NDP, NTP and ADP;
converting 1,5-AG in the sample into 1,5-AG-6P with ADP-dependent hexokinase in the presence of ADP;
dehydrogenating the formed 1,5-AG-6P with AG-6PDH in the presence of an oxidized coenzyme; and
determining the amount of the reduced coenzyme formed in the reaction mixture.

(31) A method for eliminating maltose and glucose in a sample containing maltose and glucose, in a method for the determination of 1,5-AG using an enzyme, which comprises converting maltose into glucose using enzyme system (A) capable of converting maltose into glucose and converting glucose into fructose 1,6-diphosphate using G-6P-ES, phosphohexose isomerase, 6-phosphofructokinase and NTP.

(32) The method according to (31), wherein said G-6P-ES is selected from the group consisting of (a) hexokinase and NTP, (b) glucokinase and NTP, and (c) NDP-dependent hexokinase and NDP.

(33) A reagent for the elimination of maltose and glucose in a method for the determination of 1, 5-AG using an enzyme, which comprises a reagent for the conversion of maltose into glucose comprising enzyme system (A) capable of converting maltose into glucose and a reagent for the conversion of glucose into fructose 1,6-diphosphate comprising G-6P-ES, phosphohexose isomerase, 6-phosphofructokinase and NTP.

(34) The reagent according to (33), wherein said G-6P-ES is selected from the group consisting of (a) hexokinase and NTP, (b) glucokinase and NTP, and (c) NDP-dependent hexokinase and NDP.

Brief Description of the Drawings

Fig. 1 shows a calibration curve for 1,5-AG obtained by using the reagents prepared in Example 1. The numbers on the abscissa indicate the 1,5-AG concentration ($\mu$g/ml) and the numbers on the ordinate indicate the absorbance (Abs).
Fig. 2 shows a calibration curve for 1,5-AG obtained by using the reagents prepared in Example 7. The numbers on the abscissa indicate the 1,5-AG concentration ($\mu$g/ml) and the numbers on the ordinate indicate the absorbance (Abs).
Fig. 3 shows a calibration curve for 1,5-AG obtained by using the reagents prepared in Example 11. The numbers on the abscissa indicate the 1,5-AG concentration ($\mu$g/ml) and the numbers on the ordinate indicate the absorbance (Abs).
Fig. 4 shows a calibration curve for 1,5-AG obtained by using the reagents prepared in Example 15. The numbers on the abscissa indicate the 1,5-AG concentration ($\mu$g/ml) and the numbers on the ordinate indicate the absorbance (Abs).
Fig. 5 shows a calibration curve for 1,5-AG obtained by using the reagents prepared in Example 19. The numbers on the abscissa indicate the 1,5-AG concentration ($\mu$g/ml) and the numbers on the ordinate indicate the absorbance (Abs).
Fig. 6 shows a calibration curve for 1,5-AG obtained by using the reagents prepared in Example 23. The numbers on the abscissa indicate the 1,5-AG concentration ($\mu$g/ml) and the numbers on the ordinate indicate the absorbance (Abs).
Fig. 7 shows a calibration curve for 1,5-AG obtained by using the reagents prepared in Example 27. The numbers on the abscissa indicate the 1,5-AG concentration ($\mu$g/ml) and the numbers on the ordinate indicate the absorbance (Abs).
Fig. 8 shows a calibration curve for 1,5-AG obtained by using the reagents prepared in Example 31. The numbers on the abscissa indicate the 1,5-AG concentration ($\mu$g/ml) and the numbers on the ordinate indicate the absorbance (Abs).

Detailed Description of the Invention

The present invention is applicable to the determination of 1,5-AG in any samples that may contain maltose and glucose, for example, biological samples such as blood, plasma, serum and urine.

Conversion of Maltose into Glucose

Enzyme system (A) comprises enzymes capable of converting maltose into glucose and components necessary for the enzyme reaction such as coenzymes. Examples of enzyme system (A) include (a) $\alpha$-glucosidase (EC 3.2.1.20), (b) maltose phosphorylase and inorganic phosphorus, and (c) maltose phosphorylase, inorganic phosphorus and maltose 1-epimerase.
Maltose is converted into glucose by the action of $\alpha$-glucosidase and is converted into glucose and $\beta$-glucose-1-phosphate by the action of maltose phosphorylase in the presence of inorganic phosphorus. Since $\beta$-maltose is not decomposed by maltose phosphorylase, it is preferred to convert $\beta$-maltose into $\alpha$-maltose by the action of maltose 1-epimerase.

Elimination of Glucose

Enzyme system (B) comprises enzymes capable of converting glucose into a compound which is not phosphorylated by AG-6P-ES or dehydrogenated by AG-6PDH and components necessary for the enzyme reaction such as coenzymes. Examples of enzyme system (B) include (a) G-6P-ES, phosphohexose isomerase, 6-phosphofructokinase and NTP, (b) glucose oxidase (EC 1.1.3.4), (c) glucose oxidase and mutarotase, (d) glucose oxidase and catalase, and (e) glucose oxidase, mutarotase and catalase.
G-6P-ES comprises enzymes capable of converting glucose into glucose-6-phosphate by phosphorylation and components necessary for the enzyme reaction such as coenzymes. Examples of G-6P-ES include (a) hexokinase and NTP, (b) glucokinase and NTP, and (c) NDP-dependent hexokinase and NDP. These enzymes are usually used in combination with metal ion such as magnesium ion or manganese ion. These enzymes also have the activity to convert 1,5-AG into 1,5-AG-6P and can be used also as AG-6P-ES.

When the above-mentioned enzyme system (B)-(a) is employed, glucose is converted into glucose-6-phosphate by G-6P-ES and then converted into fructose-1,6-diphosphate by the action of phosphohexose isomerase and 6-phosphofructokinase. Fructose-1,6-diphosphate, which is not reconverted into glucose or phosphorylated by AG-6P-ES or dehydrogenated by AG-6PDH, has no influence on the enzyme reaction for the determination of 1,5-AG.

When one of the above-mentioned enzyme systems (B)-(b) to (e) is employed, β-glucose is converted into glucono-1,5-lactone by oxidation with glucose oxidase in the presence of oxygen to form hydrogen peroxide. It is preferred to add mutarotase (also called "aldose 1-epimerase") to convert α-glucose in a sample into β-glucose. Further, it is preferred to add catalase to decompose hydrogen peroxide to form oxygen.

Phosphorylation of 1,5-AG

AG-6P-ES comprises enzymes capable of converting 1,5-AG into 1,5-AG-6P by phosphorylation and components necessary for the enzyme reaction such as coenzymes. Examples of AG-6P-ES include (a) hexokinase and NTP, (b) glucokinase and NTP, and (c) NDP-dependent hexokinase and NDP.

1,5-AG is phosphorylated by the enzyme system to form 1,5-AG-6P.

When the above-mentioned G-6P-ES having also the activity to phosphorylate 1,5-AG is used as a glucose 6-phosphorylating enzyme for eliminating glucose, 1,5-AG is simultaneously phosphorylated. Accordingly, the step of 1,5-AG phosphorylation can be omitted when these enzymes are used for elimination of glucose.

Dehydrogenation of 1,5-AG-6P

1,5-AG in a sample can be determined by dehydrogenating 1,5-AG-6P with AG-6PDH in the presence of an oxidized coenzyme and then determining the formed reduced coenzyme.

An example of the oxidized coenzyme is NAD(P) and an example of the reduced coenzyme is NAD(P)H.

Determination of NAD(P)H can be carried out by various known methods and the details will hereinafter be described.

The reagent for the determination of 1,5-AG according to the present invention comprises a reagent for the conversion of maltose into glucose comprising enzyme system (A) capable of converting maltose into glucose, a reagent for the elimination of glucose comprising enzyme system (B) capable of converting glucose into a compound which is not phosphorylated by AG-6P-ES or dehydrogenated by AG-6PDH, a reagent for the conversion of 1,5-AG into 1,5-AG-6P comprising AG-6P-ES, and a reagent for the dehydrogenation of 1,5-AG-6P comprising AG-6PDH and an oxidized coenzyme.

Enzyme system (A), enzyme system (B), AG-6P-ES and G-6P-ES in the above reagent have the same significances as those described in the description of the method for the 1,5-AG determination of the present invention, and examples thereof include the above-mentioned examples.

The reagent for the determination of 1,5-AG of the present invention may additionally comprise, as may be required, components necessary for the enzyme reactions, a buffer, an enzyme activity moderator, a stabilizer, a surfactant, a preservative, a chromogen, an electron carrier, a tetrazolium salt and additional enzymes.

Typical examples of the reagent kit for the determination of 1,5-AG of the present invention are as follows:

(1) a kit comprising a first container which contains a reagent for the conversion of maltose into glucose comprising enzyme system (A) capable of converting maltose into glucose, a reagent for the elimination of glucose comprising enzyme system (B) capable of converting glucose into a compound which is not phosphorylated by AG-6P-ES or dehydrogenated by AG-6PDH, and a reagent for the conversion of 1,5-AG into 1,5-AG-6P comprising AG-6P-ES, and a second container which contains a reagent for the dehydrogenation of 1,5-AG-6P comprising AG-6PDH and an oxidized coenzyme; and

(2) a kit comprising a first container which contains a reagent for the conversion of maltose into glucose comprising enzyme system (A) capable of converting maltose into glucose, and a reagent for the elimination of glucose comprising enzyme system (B) capable of converting glucose into a compound which is not phosphorylated by AG-6P-ES or dehydrogenated by AG-6PDH, and a second container which contains a reagent for the conversion of 1,5-AG into 1,5-AG-6P comprising AG-6P-ES, and a reagent for the dehydrogenation of 1,5-AG-6P comprising AG-6PDH and an oxidized coenzyme.

Enzyme system (A), enzyme system (B), AG-6P-ES and G-6P-ES in the reagent kit have the same significances as those described in the description of the method for the 1,5-AG determination of the present invention, and examples thereof include the above-mentioned examples.

When the enzymes mentioned above are employed as G-6P-ES in either reagent kit (1) or (2), AG-6P-ES can be excluded from the reagent.

The reagents in the reagent kit for the determination of 1,5-AG of the present invention may additionally comprise, as may be required, components necessary for the enzyme reactions, a buffer, an enzyme activity moderator, a stabilizer, a surfactant, a preservative, a chromogen, an electron carrier, a tetrazolium salt and additional enzymes.

In the reagent kit, the second container may contain one of the AG-6PDH and oxidized coenzyme and the first container may contain the other.

When glucose oxidase is employed for the elimination of glucose, the first container may contain NTP or NDP and the second container may contain an enzyme capable of converting 1,5-AG into 1,5-AG-6P. Examples of such enzymes include hexokinase, glucokinase and NDP-dependent hexokinase.

The reagents according to the present invention may be supplied after being freeze-dried, or after being dissolved in an aqueous medium such as water.

Detailed Description of the Determination Procedure

According to the method of the present invention, 1,5-AG can be determined by subjecting a sample to reaction for the conversion of maltose into glucose, reaction for the elimination of glucose, reaction for the phosphorylation of 1,5-AG and reaction for the dehydrogenation of 1,5-AG-6P, successively, and then determining a specific component in the obtained reaction mixture, for example, a reduced coenzyme.

Advantageously, the determination of 1,5-AG is carried out by the use of a reagent kit for the 1,5-AG determination, that is, by subjecting a sample to enzyme reactions using reagents from the first container, subjecting the resulting mixture to enzyme reaction(s) using reagent(s) from the second container, and then determining a specific component in the reaction mixture.

More advantageously, the determination of 1,5-AG is carried out by using a reagent kit further comprising components necessary for determining the specific component after completion of the dehydrogenation of 1,5-AG-6P. For example, the first container may further contain an electron carrier and the second container may further contain a tetrazolium salt.

Each enzyme reaction is carried out by adding a sample and a reagent to an appropriate aqueous medium and subjecting the resulting mixture to reaction at 10 to 50°C for 1 to 30 minutes, preferably at 20 to 40°C for 2 to 10 minutes, if necessary in the presence of a magnesium salt, an enzyme activity moderator, a stabilizer, a surfactant, etc.

Either the oxidized coenzyme or AG-6PDH used for the 1,5-AG determination may be present in the step of glucose elimination, so far as it does not affect the reaction for elimination of glucose.

The following enzymes used in the present invention are all known ones and are commercially available or easily producible: α-glucosidase, maltose 1-epimerase, maltose phosphorylase, NDP-dependent hexokinase such as ADP-dependent hexokinase, hexokinase, phosphohexose isomerase, 6-phosphofructokinase, mutarotase, glucose oxidase, catalase, 1,5-anhydroglucitol-6-phosphate dehydrogenase, diaphorase, NAD(P)H oxidase and peroxidase.

For example, as the ADP-dependent hexokinase, those derived from Thermococcus litoralis and Pyrococcus furiosus are available from Asahi Kasei Corporation. Phosphohexose isomerase derived from Bacillus stearothermophilus is available from Unitika Ltd. and 6-phosphofructokinase derived from Bacillus stearothermophilus is also available from Unitika Ltd.

As the AG-6PDH, the one derived from Escherichia coli DH1 (ATCC 33849) can be prepared according to the method described in Japanese Published Unexamined Patent Application No. 84953/98.

The concentrations of the enzymes to be used in the reaction mixture are as follows: α-glucosidase, 0.5 to 100 U/ml, preferably 1 to 50 U/ml; maltose 1-epimerase, 0.5 to 100 U/ml, preferably 1 to 50 U/ml; maltose phosphorylase, 1 to 300 U/ml, preferably 3 to 100 U/ml; NDP-dependent hexokinase, 0.5 to 100 U/ml, preferably 1 to 50 U/ml; hexokinase, 1.0 to 300 U/ml, preferably 3 to 100 U/ml; phosphohexose isomerase, 1 to 300 U/ml, preferably 3 to 100 U/ml; 6-phosphofructokinase, 1 to 300 U/ml, preferably 3 to 100 U/ml; mutarotase, 0.5 to 100 U/ml, preferably 1 to 50 U/ml; glucose oxidase, 10 to 2000 U/ml, preferably 20 to 1000 U/ml; catalase, 10 to 2000 U/ml, preferably 20 to 1000 U/ml; and 1,5-anhydroglucitol-6-phosphate dehydrogenase, 0.5 to 100 U/ml, preferably 1 to 50 U/ml.

Examples of the oxidized coenzymes include oxidized nicotinamide adenine dinucleotide (NAD), oxidized nicotinamide adenine dinucleotide phosphate (NADP), thio NAD and thio NADP.

Examples of NTP (nucleoside triphosphate) include adenosine triphosphate (ATP), guanosine triphosphate, cytidine triphosphate, thiamine triphosphate, uridine triphosphate and inosine triphosphate. Preferred is ATP.

Examples of NDP (nucleoside diphosphate) include adenosine diphosphate (ADP), guanosine diphosphate, cytidine diphosphate, thiamine diphosphate, uridine diphosphate and inosine diphosphate. Preferred is ADP.

Examples of inorganic phosphorus include phosphoric acid and phosphates (e.g., sodium phosphate, potassium phosphate and magnesium phosphate).

The oxidized coenzyme, NTP, NDP and inorganic phosphorus are respectively used at a concentration of 0.01 to 100 mmol/l, preferably 0.1 to 50 mmol/l in the reaction mixture.

Suitable aqueous media include water, distilled water, and water-containing liquids such as buffers and physiological saline. Buffers are preferably used.

Examples of the buffers are lactate buffer, citrate buffer, acetate buffer, succinate buffer, phthalate buffer, phosphate buffer, triethanolamine buffer, diethanolamine buffer, lysine buffer, barbital buffer, tris(hydroxymethyl)aminomethane buffer, imidazole buffer, malate buffer, oxalate buffer, glycine buffer, borate buffer, carbonate buffer, Good's buffer, N-(2-acetamido)imino diacetate buffer (hereinafter abbreviated as ADA buffer), and N-tris(hydroxymethyl)methyl-3-aminopropane sulfonate buffer (hereinafter abbreviated as TAPS buffer). They are used at a concentration of 0.1 to 1000 mmol/l, preferably 1 to 500 mmol/l in the reaction mixture.

Examples of the enzyme activity moderators include metal chelating agents such as 1,10-phenanthroline, and metal ions such as magnesium ion and manganese ion. An example of the source of magnesium ion is magnesium acetate.

Examples of the stabilizers include metal chelates such as ethylenediaminetetraacetic acid, polysaccharides such as soluble starch and derivatives thereof, proteins such as albumin and globulin, and water-soluble high-molecular weight compounds such as polyethylene glycol.

Examples of the surfactants are polyoxyethylene octylphenyl ether (Nonion HS-210, NOF Corporation), 3-[(3-chloramidepropyl)dimethylamino]propanesulfonic acid, Triton X-100 and sodium dodecyl sulfate.

In the above method for the 1,5-AG determination, the determination of a component consumed or formed by the reaction (e.g., reduced coenzyme) can be carried out, for example, by directly determining the reduced coenzyme formed by the reaction by measuring the absorbance of the reaction mixture at 340 nm, or by converting the reduced coenzyme into another substance and then determining the resulting substance.

An example of the method of determining a reduced coenzyme via another substance is a method which comprises subjecting the formed reduced coenzyme such as NAD(P)H to reaction with a tetrazolium salt in the presence of an electron carrier such as diaphorase (EC 1.6.99.1) or 1-methoxy-5-methylphenazine methosulfate, and measuring the absorbance of the reaction mixture colored by the formed formazan pigment at 438 nm.

There is no specific restriction as to the tetrazolium salts so far as they can be used as chromogens in this method. Preferred are those having high molecular extinction coefficient, from the viewpoint of sensitivity, for example, indonitro tetrazolium (INT), nitro blue tetrazolium (NBT), 2-(4-iodophenyl)-3-(4-nitrophenyl)-5-(2,4-disulfonyl)-2H-tetrazolium monosodium salt (hereinafter referred to as WST-1), 2-(4-iodophenyl)-3-(2,4-dinitrophenyl)-5-(2,4-disulfenyl)-2H-tetrazolium monosodium salt (hereinafter referred to as WST-3), 3,3'-[3,3'-dimethoxy-(1,1'-biphenyl)-4,4'-diyl]-bis[2-(4-nitrophenyl)-5-phenyl-2H tetrazolium chloride] (NTB) and 3-(4,5-dimethylthiazole-2-phenyl)-5-(3-carboxymethoxyphenyl)-2-(4-sulfophenyl)-2H-tetrazolium salt (MTS). They are used at a concentration of 0.01 to 50 mmol/l, preferably 0.05 to 10 mmol/l in the reaction mixture.

Suitable electron carriers include phenazine methosulfate, 1-methoxy-5-methylphenazine methosulfate, and Meldola's Blue. They are used at a concentration of 0.01 to 50 mM, preferably 0.05 to 10 mM in the reaction mixture.

Diaphorase is also useful as an electron carrier. An example of the diaphorase is the one derived from Bacillus megaterium, which is commercially available. The concentration of diaphorase in the reaction mixture is 0.01 to 100 U/l, preferably 0.05 to 50 U/l.

The reaction is carried out at 10 to 50°C for 1 to 30 minutes, preferably 2 to 10 minutes.

Another example of the method of determining a reduced coenzyme via another substance is a method which comprises oxidizing the reduced coenzyme such as NAD(P)H by the action of NAD(P)H oxidase to form hydrogen peroxide, subjecting the formed hydrogen peroxide to reaction with a chromogen in the presence of peroxidase (EC 1.11.1.7), and measuring the absorbance of the reaction mixture colored by the formed pigment.

NAD(P)H oxidase is used at a concentration of 0.5 to 100 U/ml, preferably 1 to 50 U/ml, and peroxidase is used at a concentration of 0.5 to 100 U/ml, preferably 1 to 50 U/ml in the reaction mixture.

As the chromogen, chromogens used in combination with 4-aminoantipyrine or the like may be used, but those which can be used alone to produce pigments are preferred.

Examples of the chromogens which can be used alone are bis[3-bis(4-chlorophenyl)-methyl-4-dimethylaminophenyl]amine (BCMA), bis[3-bis(4-chlorophenyl)-methyl-4-carboxyethylaminophenyl]amine, 10-N-methylcarbamoyl-3,7-dimethylamino-10H-phenothiazine (MCDP) and 10-N-carboxymethylcarbamoyl-3,7-dimethylamino-10H-phenothiazine (CCAP).

An example of the chromogens to be used in combination with 4-aminoantipyrine is N-ethyl-N-(3-methylphenyl)-N'-succinylethylenediamine (EMSE).

The reaction is carried out at 10 to 50°C for 1 to 30 minutes, preferably 2 to 10 minutes.

Certain embodiments of the present invention are illustrated in the following examples. These examples are not to be construed as limiting the scope of the invention.

The abbreviations used in the examples are as follows.

Compounds

1,5-AG: 1,5-Anhydroglucitol
F-1,6-2P: Fructose-1,6-diphosphate

Enzymes

α-GH: α-Glucosidase
AG-6PDH: 1,5-Anhydroglucitol-6-phosphate dehydrogenase
ADP-HK: ADP-dependent hexokinase
DIP: Diaphorase
GOD: Glucose oxidase
HK: Hexokinase
MER: Maltose 1-epimerase
MP: Maltose phosphorylase
PFK: 6-Phosphofructokinase
PGI: Phosphohexose isomerase

Coenzymes

ADP: Adenosine diphosphate
ATP: Adenosine triphosphate
NAD: Nicotinamide adenine dinucleotide
NADH: Reduced nicotinamide adenine dinucleotide
NADP: Nicotinamide adenine dinucleotide phosphate
NADPH: Reduced nicotinamide adenine dinucleotide phosphate
NDP: Nucleoside diphosphate
NTP: Nucleoside triphosphate

Example 1

Reagents for the determination of 1,5-AG having the following compositions were prepared.

| Reagent 1 | |
| --- | --- |
| ADA buffer (pH 7.0) | 20 mmol/l |
| Magnesium acetate | 8 mmol/l |
| NADP (Oriental Yeast Co., Ltd.) | 10 mmol/l |
| ADP (Oriental Yeast Co., Ltd.) | 0.5 mmol/l |
| ATP (Kyowa Hakko Kogyo Co., Ltd.) | 10 mmol/l |
| PGI (derived from Bacillus stearothermophilus, Unitika Ltd.) | 50 KU/l |
| PFK (derived from Bacillus stearothermophilus, Unitika Ltd.) | 50 KU/l |
| DIP (derived from Clostridium sp., Toyobo Co., Ltd.) | 3 KU/l |
| ADP-HK (derived from Thermococcus litoralis, Asahi Kasei Corporation) | 10 KU/l |

(continued)

| Reagent 1 | |
|---|---|
| α-GH (derived from Bacillus stearothermophilus, Toyobo Co., Ltd.) | 5 KU/l |

| Reagent 2 | |
|---|---|
| TAPS buffer (pH 8.5) | 200 mmol/l |
| WST-1 (Dojindo Laboratories) | 0.6 mmol/l |
| AG-6PDH [derived from E. coli DF1 (ATCC 33849), Asahi Kasei Corporation] | 50 KU/l |

Comparative Example 1

Reagents for the determination of 1,5-AG having the following compositions were prepared.

| Reagent 1 | |
|---|---|
| ADA buffer (pH 7.0) | 20 mmol/l |
| Magnesium acetate | 8 mmol/l |
| NADP (Oriental Yeast Co., Ltd.) | 10 mmol/l |
| ADP (Oriental Yeast Co., Ltd.) | 0.5 mmol/l |
| ATP (Kyowa Hakko Kogyo Co., Ltd.) | 10 mmol/l |
| PGI (derived from Bacillus stearothermophilus, Unitika Ltd.) | 50 KU/l |
| PFK (derived from Bacillus stearothermophilus, Unitika Ltd.) | 50 KU/l |
| DIP (derived from Clostridium sp., Toyobo Co., Ltd.) | 3 KU/l |
| ADP-HK (derived from Thermococcus litoralis, Asahi Kasei Corporation) | 10 KU/l |

| Reagent 2 | |
|---|---|
| TAPS buffer (pH 8.5) | 200 mmol/l |
| WST-1 (Dojindo Laboratories) | 0.6 mmol/l |
| AG-6PDH [derived from E. coli DF1 (ATCC 33849), Asahi Kasei Corporation] | 50 KU/l |

Example 2

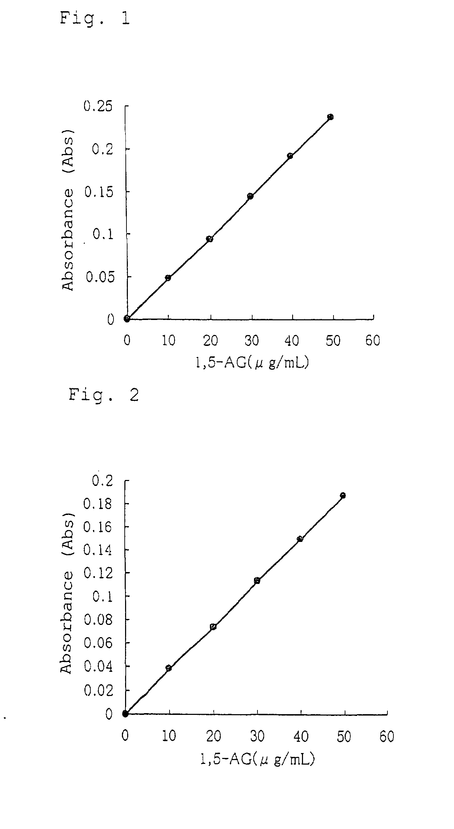

1,5-AG was dissolved in purified water to prepare standard solutions respectively having the 1,5-AG concentrations of 0, 10, 20, 30, 40 and 50 µg/ml. To 0.075 ml of each of the solutions was added 2.25 ml of reagent 1 prepared in Example 1, followed by incubation at 37°C for 5 minutes. After 0.75 ml of reagent 2 prepared in Example 1 was added to each mixture, the reaction was carried out for 5 minutes. The absorbance was measured at 438 nm and a calibration curve was obtained. The result is shown in Fig. 1.
The calibration curve obtained by using the reagents prepared in Example 1 showed almost a linear relationship between 1,5-AG concentration and absorbance.

Example 3

Reagent 1 prepared in Example 1 was poured into test tubes in 2.25 ml portions. To the test tubes were respectively added 0.075 ml each of (a) purified water (blank), (b) a solution containing 25 µg/ml 1,5-AG, (c) a solution containing 1000 mg/dl maltose, and (d) a solution containing 25 µg/ml 1,5-AG and 1000 mg/dl maltose, followed by incubation at 37°C for 5 minutes. After 0.75 ml of reagent 2 prepared in Example 1 was added to each mixture, the reaction was carried out for 5 minutes and the absorbance was measured at 438 nm. The same procedure was repeated using the reagents prepared in Comparative Example 1. The results are shown in Table 1.

Table 1

| Sample | Absorbance (Abs) | |
|---|---|---|
| | Reagents of Example 1 | Reagents of Comp. Example 1 |
| (a) Purified water (blank) | 0.010 | 0.010 |
| (b) 25 μg/ml 1,5-AG | 0.125 | 0.125 |
| (c) 1000 mg/dl Maltose | 0.011 | 0.141 |
| (c)-(a) Reaction of maltose | 0.001 | 0.131 |
| (d) 1,5-AG + maltose | 0.126 | 0.256 |

As shown in Table 1, when the reagents of Example 1 were used, the value of (c) closely agreed with that of (a) and the value of (c)-(a) was close to 0, indicating that maltose contained in solution (c) was eliminated. Accordingly, both solution (b) containing 1,5-AG (25 μg/ml) alone and solution (d) containing 1,5-AG (25 μg/ml) plus maltose (1000 mg/dl) showed almost the same absorbances. The utility of the method according to the invention was thus proved.

On the other hand, when the reagents of Comparative Example 1 which do not contain α-GH were used, it is apparent from the value of (c) that the presence of maltose affected the 1,5-AG determination and the concentration of 1,5-AG in the solution containing maltose could not be measured accurately.

Example 4

Reagent 1 prepared in Example 1 was poured into test tubes in 2.25 ml portions. To the test tubes were respectively added 0.075 ml each of (a) purified water (blank), (b) a solution containing 1000 mg/dl glucose, and (c) a solution containing 1000 mg/dl glucose and 500 mg/dl maltose, followed by incubation at 37°C for 5 minutes. After 0.75 ml of reagent 2 prepared in Example 1 was added to each mixture, the reaction was carried out for 5 minutes and the absorbance was measured at 438 nm. The same procedure was repeated using the reagents prepared in Comparative Example 1. The results are shown in Table 2.

Table 2

| Sample | Absorbance (Abs) | |
|---|---|---|
| | Reagents of Example 1 | Reagents of Comp. Example 1 |
| (a) Purified water (blank) | 0.010 | 0.010 |
| (b) 1000 mg/dl Glucose | 0.012 | 0.010 |
| (c) 1000 mg/dl Glucose + 500 mg/dl maltose | 0.020 | 0.101 |
| (c)-(b) Reaction of maltose | 0.008 | 0.091 |

As shown in Table 2, when the reagents of Example 1 were used, the values of (b) and (c) closely agreed with that of (a) and the values of (b)-(a) and (c)-(a) were close to 0, indicating that glucose (1000 mg/dl) contained in solution (b) and glucose (1000 mg/dl) and maltose (500 mg/dl) in solution (c) were eliminated. Thus, it is demonstrated that 1,5-AG concentration can be measured without being affected by glucose and maltose in a sample by using the reagents according to the present invention.

On the other hand, when the reagents of Comparative Example 1 which do not contain α-GH are used, it is apparent from the value of (c) that the presence of maltose will affect the 1,5-AG determination and the concentration of 1,5-AG in a solution containing maltose can not be measured accurately.

Example 5

Samples respectively containing 25 μg/ml 1,5-AG plus maltose at the concentrations indicated in Table 3 were prepared. To 0.075 ml of each of the samples was added 2.25 ml of reagent 1 prepared in Example 1, followed by incubation at 37°C for 5 minutes. After 0.75 ml of reagent 2 prepared in Example 1 was added to each mixture, the reaction was carried out for 5 minutes. The absorbance was measured at 438 nm, and 1,5-AG was determined using the calibration curve obtained in Example 2. Separately, 1,5-AG in the above samples was determined in the same manner using the reagents prepared in Comparative Example 1. The results are shown in Table 3.

Table 3

| Maltose (mg/dl) | Reagents of Example 1 | | Reagents of Comp. Ex. 1 | |
|---|---|---|---|---|
| | 1,5-AG value (µg/ml) | Maltose effect (%) | 1,5-AG value (µg/ml) | Maltose effect (%) |
| 0 | 25.2 | 0 | 25.2 | 0 |
| 200 | 25.3 | 0.4 | 29.4 | 16.7 |
| 400 | 25.5 | 1.2 | 33.3 | 32.1 |
| 600 | 25.3 | 0.4 | 38.4 | 52.4 |
| 800 | 25.4 | 0.8 | 43.6 | 73.0 |
| 1000 | 25.5 | 1.2 | 46.4 | 84.1 |

It was demonstrated that the concentration of 1,5-AG in a sample containing maltose even at a high concentration (1000 mg/dl) could be accurately measured by using the reagents prepared in Example 1. The maximal effect rate of maltose was 1.2%, and the obtained 1,5-AG values were clinically satisfactory.
The maltose effect rate can be calculated by the following equation.

$$\text{Effect rate (\%)} = 100 \times |A-B|/A$$

A: 1,5-AG value obtained for a sample containing maltose
B: 1,5-AG value obtained for a sample containing no maltose

On the other hand, when the reagents prepared in Comparative Example 1 were used, 1,5-AG could not be accurately measured by the effect of maltose.

Example 6

Samples respectively containing 1000 mg/dl glucose plus maltose at the concentrations indicated in Table 4 were prepared. To 0.075 ml of each of the samples was added 2.25 ml of reagent 1 prepared in Example 1, followed by incubation at 37°C for 5 minutes. After 0.75 ml of reagent 2 prepared in Example 1 was added to each mixture, the reaction was carried out for 5 minutes. The absorbance was measured at 438 nm, and 1,5-AG was determined using the calibration curve obtained in Example 2. Separately, 1,5-AG in the above samples was determined in the same manner using the reagents prepared in Comparative Example 1. The results are shown in Table 4.

Table 4

| Maltose (mg/dl) | 1,5-AG Value (µg/ml) | |
|---|---|---|
| | Reagents of Example 1 | Reagents of Comp. Example 1 |
| 0 | 0.2 | 0.0 |
| 100 | 0.6 | 3.4 |
| 200 | 1.0 | 6.9 |
| 300 | 1.4 | 10.2 |
| 400 | 1.8 | 14.3 |
| 500 | 2.1 | 19.1 |

It is apparent from the results in Table 4 that the reagents of Example 1 are capable of eliminating glucose and maltose in a sample and 1,5-AG determination using them is little affected by glucose and maltose, whereas 1,5-AG determination using the reagents of Comparative Example 1 is considerably affected by maltose in a sample.

Example 7

Reagents for the determination of 1,5-AG having the following compositions were prepared.

| Reagent 1 | |
|---|---|
| ADA buffer (pH 7.0) | 20 mmol/l |
| Magnesium acetate | 8 mmol/l |
| NADP (Oriental Yeast Co., Ltd.) | 10 mmol/l |
| ADP (Oriental Yeast Co., Ltd.) | 0.5 mmol/l |
| ATP (Kyowa Hakko Kogyo Co., Ltd.) | 10 mmol/l |
| PGI (derived from Bacillus stearothermophilus, Unitika Ltd.) | 50 KU/l |
| PFK (derived from Bacillus stearothermophilus, Unitika Ltd.) | 50 KU/l |
| DIP (derived from Clostridium sp., Toyobo Co., Ltd.) | 3 KU/l |
| ADP-HK (derived from Thermococcus litoralis, Asahi Kasei Corporation) | 10 KU/l |
| MER (derived from Lactobacillus brevis, Kikkoman Corporation) | 20 KU/l |
| MP (derived from recombinant E. coli, Kikkoman Corporation) | 12 KU/l |

| Reagent 2 | |
|---|---|
| TAPS buffer (pH 8.5) | 200 mmol/l |
| WST-1 (Dojindo Laboratories) | 0.6 mmol/l |
| AG-6PDH [derived from E. coli DF1 (ATCC 33849), Asahi Kasei Corporation] | 50 KU/l |

Example 8

1,5-AG was dissolved in purified water to prepare standard solutions respectively having the 1,5-AG concentrations of 0, 10, 20, 30, 40 and 50 μg/ml. To 0.075 ml of each of the solutions was added 2.25 ml of reagent 1 prepared in Example 7, followed by incubation at 37°C for 5 minutes. After 0.75 ml of reagent 2 prepared in Example 7 was added to each mixture, the reaction was carried out for 5 minutes. The absorbance was measured at 438 nm and a calibration curve was obtained. The result is shown in Fig. 2.
The calibration curve obtained by using the reagents prepared in Example 7 showed almost a linear relationship between 1,5-AG concentration and absorbance.

Example 9

Reagent 1 prepared in Example 7 was poured into test tubes in 2.25 ml portions. To the test tubes were respectively added 0.075 ml each of (a) purified water (blank), (b) a solution containing 25 μg/ml 1,5-AG, (c) a solution containing 1000 mg/dl maltose, and (d) a solution containing 25 μg/ml 1,5-AG and 1000 mg/dl maltose, followed by incubation at 37°C for 5 minutes. After 0.75 ml of reagent 2 prepared in Example 7 was added to each mixture, the reaction was carried out for 5 minutes and the absorbance was measured at 438 nm. The results are shown in Table 5.

Table 5

| Sample | Absorbance (Abs) |
|---|---|
| (a) Purified water (blank) | 0.016 |
| (b) 25 μg/ml 1,5-AG | 0.105 |
| (c) 1000 mg/dl Maltose | 0.016 |

Table 5   (continued)

| Sample | Absorbance (Abs) |
|---|---|
| (c)-(a) Reaction of maltose | 0.000 |
| (d) 1,5-AG + maltose | 0.105 |

As shown in Table 5, the value of (c) agreed with that of (a), indicating that maltose contained in solution (c) was eliminated by the method of the invention. Further, the value of (b) agreed with that of (d). The utility of the method according to the invention was thus proved.

Example 10

Samples respectively containing 25 μg/ml 1,5-AG plus maltose at the concentrations indicated in Table 6 were prepared. To 0.075 ml of each of the samples was added 2.25 ml of reagent 1 prepared in Example 7, followed by incubation at 37°C for 5 minutes. After 0.75 ml of reagent 2 prepared in Example 7 was added to each mixture, the reaction was carried out for 5 minutes. The absorbance was measured at 438 nm, and 1,5-AG was determined using the calibration curve obtained in Example 8. The results are shown in Table 6.

Table 6

| Maltose (mg/dl) | 1,5-AG value (μg/ml) | Maltose effect (%) |
|---|---|---|
| 0 | 25.4 | 0 |
| 200 | 25.6 | 0.8 |
| 400 | 25.5 | 0.4 |
| 600 | 25.5 | 0.4 |
| 800 | 25.6 | 0.8 |
| 1000 | 25.8 | 1.6 |

It was demonstrated that the concentration of 1,5-AG in a sample containing maltose even at a high concentration (1000 mg/dl) could be accurately measured by using the reagents prepared in Example 7. The maximal effect rate of maltose was 1.6%, and the obtained 1,5-AG values were clinically satisfactory.

Example 11

Reagents for the determination of 1,5-AG having the following compositions were prepared.

| Reagent 1 | |
|---|---|
| ADA buffer (pH 7.0) | 20 mmol/l |
| Magnesium acetate | 8 mmol/l |
| NADP (Oriental Yeast Co., Ltd.) | 10 mmol/l |
| ATP (Kyowa Hakko Kogyo Co., Ltd.) | 10 mmol/l |
| PGI (derived from Bacillus stearothermophilus, Unitika Ltd.) | 50 KU/l |
| PFK (derived from Bacillus stearothermophilus, Unitika Ltd.) | 50 KU/l |
| DIP (derived from Clostridium sp., Toyobo Co., Ltd.) | 3 KU/l |
| HK (derived from Thermococcus litoralis, Asahi Kasei Corporation) | 100 KU/l |
| α-GH (derived from Bacillus stearothermophilus, Toyobo Co., Ltd.) | 5 KU/l |

| Reagent 2 | |
|---|---|
| APS buffer (pH 8.5) | 200 mmol/l |
| WST-1 (Dojindo Laboratories) | 0.6 mmol/l |
| AG-6PDH [derived from E. coli DF1 (ATCC 33849), Asahi Kasei Corporation] | 50 KU/l |

Example 12

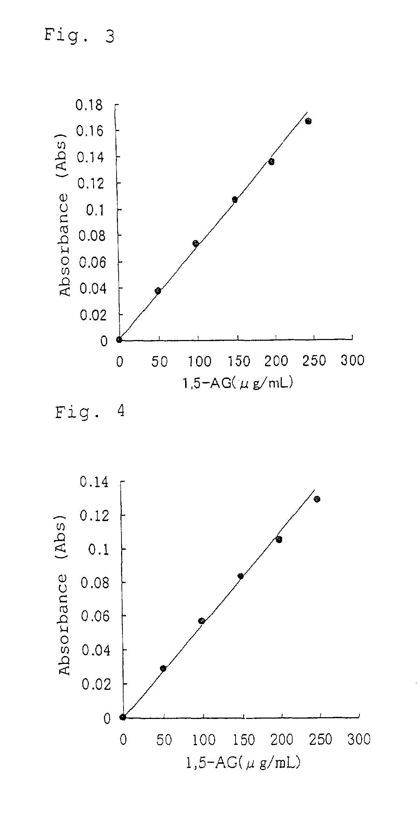

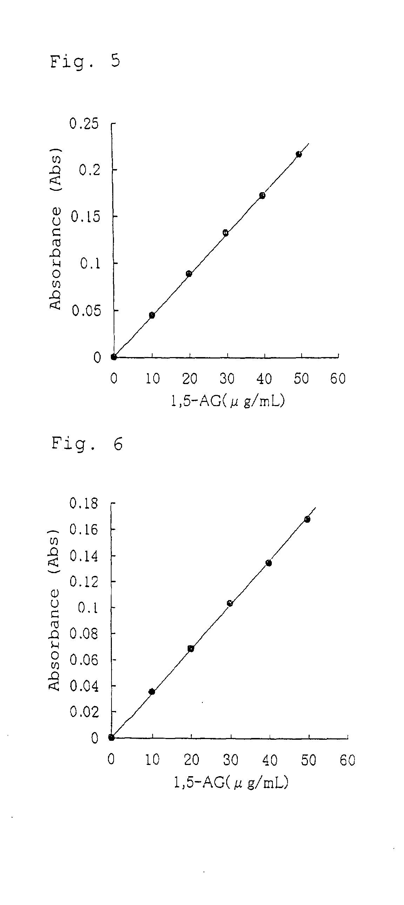

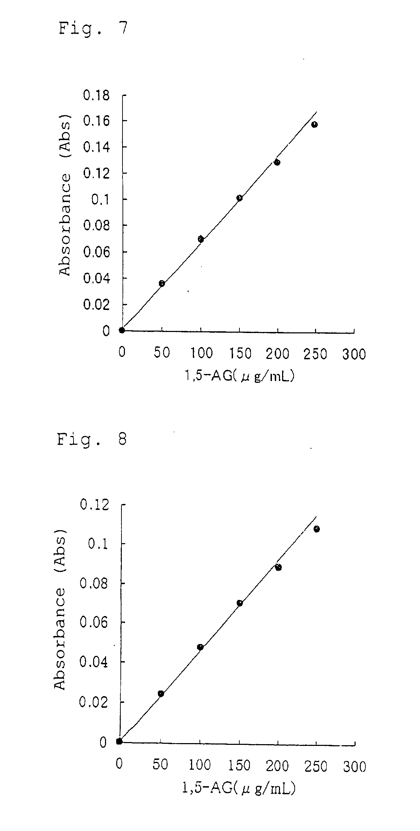

1,5-AG was dissolved in purified water to prepare standard solutions respectively having the 1,5-AG concentrations of 0, 50, 100, 150, 200 and 250 μg/ml. To 0.075 ml of each of the solutions was added 2.25 ml of reagent 1 prepared in Example 11, followed by incubation at 37°C for 5 minutes. After 0.75 ml of reagent 2 prepared in Example 11 was added to each mixture, the reaction was carried out for 5 minutes. The absorbance was measured at 438 nm and a calibration curve was obtained. The result is shown in Fig. 3.

The calibration curve obtained by using the reagents prepared in Example 11 showed almost a linear relationship between 1,5-AG concentration and absorbance.

Example 13

Reagent 1 prepared in Example 11 was poured into test tubes in 2.25 ml portions. To the test tubes were respectively added 0.075 ml each of (a) purified water (blank), (b) a solution containing 250 μg/ml 1,5-AG, (c) a solution containing 1000 mg/dl maltose, and (d) a solution containing 250 μg/ml 1,5-AG and 1000 mg/dl maltose, followed by incubation at 37°C for 5 minutes. After 0.75 ml of reagent 2 prepared in Example 11 was added to each mixture, the reaction was carried out for 5 minutes and the absorbance was measured at 438 nm. The results are shown in Table 7.

Table 7

| Sample | Absorbance (Abs) |
|---|---|
| (a) Purified water (blank) | 0.288 |
| (b) 250 μg/ml 1,5-AG | 0.453 |
| (c) 1000 mg/dl Maltose | 0.289 |
| (c)-(a) Reaction of maltose | 0.001 |
| (d) 1,5-AG + maltose | 0.454 |

As shown in Table 7, the value of (c) closely agreed with that of (a) and the value of (c)-(a) was close to 0, indicating that maltose contained in solution (c) was eliminated by the method of the invention using the reagents prepared in Example 11. Accordingly, both solution (b) containing 1,5-AG (250 μg/ml) alone and solution (d) containing 1,5-AG (250 μg/ml) plus maltose (1000 mg/dl) showed almost the same absorbances. The utility of the method according to the invention was thus proved.

Example 14

Samples respectively containing 250 μg/ml 1,5-AG plus maltose at the concentrations indicated in Table 8 were prepared. To 0.075 ml of each of the samples was added 2.25 ml of reagent 1 prepared in Example 11, followed by incubation at 37°C for 5 minutes. After 0.75 ml of reagent 2 prepared in Example 11 was added to each mixture, the reaction was carried out for 5 minutes. The absorbance was measured at 438 nm, and 1,5-AG was determined using the calibration curve obtained in Example 12. The results are shown in Table 8.

Table 8

| Maltose (mg/dl) | 1,5-AG value (μg/ml) | Maltose effect (%) |
|---|---|---|
| 0 | 251.5 | 0 |
| 200 | 251.8 | 0.1 |
| 400 | 252.2 | 0.3 |

Table 8 (continued)

| Maltose (mg/dl) | 1,5-AG value (μg/ml) | Maltose effect (%) |
|---|---|---|
| 600 | 252.6 | 0.4 |
| 800 | 252.8 | 0.5 |
| 1000 | 252.5 | 0.4 |

It was demonstrated that the concentration of 1,5-AG in a sample containing maltose even at a high concentration (1000 mg/dl) could be accurately measured by using the reagents prepared in Example 11. The maximal effect rate of maltose was 0.5%, and the obtained 1,5-AG values were clinically satisfactory.

Example 15

Reagents for the determination of 1,5-AG having the following compositions were prepared.

| Reagent 1 | |
|---|---|
| ADA buffer (pH 7.0) | 20 mmol/l |
| Magnesium acetate | 8 mmol/l |
| NADP (Oriental Yeast Co., Ltd.) | 10 mmol/l |
| ATP (Kyowa Hakko Kogyo Co., Ltd.) | 10 mmol/l |
| PGI (derived from Bacillus stearothermophilus, Unitika Ltd.) | 50 KU/l |
| PFK (derived from Bacillus stearothermophilus, Unitika Ltd.) | 50 KU/l |
| DIP (derived from Clostridium sp., Toyobo Co., Ltd.) | 3 KU/l |
| HK (derived from Thermococcus litoralis, Asahi Kasei Corporation) | 100 KU/l |
| MER (derived from Lactobacillus brevis, Kikkoman Corporation) | 20 KU/l |
| MP (derived from recombinant E. coli, Kikkoman Corporation) | 12 KU/l |

| Reagent 2 | |
|---|---|
| TAPS buffer (pH 8.5) | 200 mmol/l |
| WST-1 (Dojindo Laboratories) | 0.6 mmol/l |
| AG-6PDH [derived from E. coli DF1 (ATCC 33849), Asahi Kasei Corporation] | 50 KU/l |

Example 16

1,5-AG was dissolved in purified water to prepare standard solutions respectively having the 1,5-AG concentrations of 0, 50, 100, 150, 200 and 250 μg/ml. To 0.075 ml of each of the solutions was added 2.25 ml of reagent 1 prepared in Example 15, followed by incubation at 37°C for 5 minutes. After 0.75 ml of reagent 2 prepared in Example 15 was added to each mixture, the reaction was carried out for 5 minutes. The absorbance was measured at 438 nm and a calibration curve was obtained. The result is shown in Fig. 4.
The calibration curve obtained by using the reagents prepared in Example 15 showed almost a linear relationship between 1,5-AG concentration and absorbance.

Example 17

Reagent 1 prepared in Example 15 was poured into test tubes in 2.25 ml portions. To the test tubes were respectively added 0.075 ml each of (a) purified water (blank), (b) a solution containing 250 μg/ml 1,5-AG, (c) a solution containing 1000 mg/dl maltose, and (d) a solution containing 250 µg/ml 1,5-AG and 1000 mg/dl maltose, followed by incubation at 37°C for 5 minutes. After 0.75 ml of reagent 2 prepared in Example 15 was added to each mixture, the reaction was carried out for 5 minutes and the absorbance was measured at 438 nm. The results are shown in Table 9.

Table 9

| Sample | Absorbance (Abs) |
|---|---|
| (a) Purified water (blank) | 0.293 |
| (b) 250 µg/ml 1,5-AG | 0.421 |
| (c) 1000 mg/dl Maltose | 0.293 |
| (c)-(a) Reaction of maltose | 0.000 |
| (d) 1,5-AG + maltose | 0.421 |

As shown in Table 9, the value of (c) agreed with that of (a), indicating that maltose contained in solution (c) was eliminated by the method of the invention. Further, the value of (b) agreed with that of (d). The utility of the method according to the invention was thus proved.

Example 18

Samples respectively containing 250 µg/ml 1,5-AG plus maltose at the concentrations indicated in Table 10 were prepared. To 0.075 ml of each of the samples was added 2.25 ml of reagent 1 prepared in Example 15, followed by incubation at 37°C for 5 minutes. After 0.75 ml of reagent 2 prepared in Example 15 was added to each mixture, the reaction was carried out for 5 minutes. The absorbance was measured at 438 nm, and 1,5-AG was determined using the calibration curve obtained in Example 16. The results are shown in Table 10.

Table 10

| Maltose (mg/dl) | 1,5-AG value (µg/ml) | Maltose effect (%) |
|---|---|---|
| 0 | 251.7 | 0 |
| 200 | 251.9 | 0.1 |
| 400 | 252.4 | 0.3 |
| 600 | 251.9 | 0.1 |
| 800 | 252.1 | 0.2 |
| 1000 | 252.4 | 0.3 |

It was demonstrated that the concentration of 1,5-AG in a sample containing maltose even at a high concentration (1000 mg/dl) could be accurately measured by using the reagents prepared in Example 15. The maximal effect rate of maltose was 0.3%, and the obtained 1,5-AG values were clinically satisfactory.

**Example 19**

Reagents for the determination of 1,5-AG having the following compositions were prepared.

| Reagent 1 | |
|---|---|
| ADA buffer (pH 7.0) | 20 mmol/l |
| Magnesium acetate | 8 mmol/l |
| NADP (Oriental Yeast Co., Ltd.) | 10 mmol/l |
| ADP (Oriental Yeast Co., Ltd.) | 0.5 mmol/l |
| DIP (derived from Clostridium sp., Toyobo Co., Ltd.) | 3 KU/l |
| α-GH (derived from Bacillus stearothermophilus, Toyobo Co., Ltd.) | 5 KU/l |

(continued)

| Reagent 1 | |
|---|---|
| Mutarotase (derived from pig kidney, Oriental Yeast Co., Ltd.) | 10 KU/l |
| GOD (derived from Aspergillus sp., Toyobo Co., Ltd.) | 300 KU/l |
| Catalase (derived from bovine liver, Sigma Chemical Co.) | 100 KU/l |

| Reagent 2 | |
|---|---|
| TAPS buffer (pH 8.5) | 200 mmol/l |
| WST-1 (Dojindo Laboratories) | 0.6 mmol/l |
| ADP-HK (derived from Thermococcus litoralis, Asahi Kasei Corporation) | 40 KU/l |
| AG-6PDH [derived from E. coli DF1 (ATCC 33849), Asahi Kasei Corporation] | 50 KU/l |

Example 20

1,5-AG was dissolved in purified water to prepare standard solutions respectively having the 1,5-AG concentrations of 0, 10, 20, 30, 40 and 50 μg/ml. To 0.075 ml of each of the solutions was added 2.25 ml of reagent 1 prepared in Example 19, followed by incubation at 37°C for 5 minutes. After 0.75 ml of reagent 2 prepared in Example 19 was added to each mixture, the reaction was carried out for 5 minutes. The absorbance was measured at 438 nm and a calibration curve was obtained. The result is shown in Fig. 5.

The calibration curve obtained by using the reagents prepared in Example 19 showed almost a linear relationship between 1,5-AG concentration and absorbance.

Example 21

Reagent 1 prepared in Example 19 was poured into test tubes in 2.25 ml portions. To the test tubes were respectively added 0.075 ml each of (a) purified water (blank), (b) a solution containing 25 μg/ml 1,5-AG, (c) a solution containing 400 mg/dl maltose, and (d) a solution containing 25 μg/ml 1,5-AG and 400 mg/dl maltose, followed by incubation at 37°C for 5 minutes. After 0.75 ml of reagent 2 prepared in Example 19 was added to each mixture, the reaction was carried out for 5 minutes and the absorbance was measured at 438 nm. The results are shown in Table 11.

Table 11

| Sample | Absorbance (Abs) |
|---|---|
| (a) Purified water (blank) | 0.025 |
| (b) 25 μg/ml 1,5-AG | 0.132 |
| (c) 400 mg/dl Maltose | 0.024 |
| (c)-(a) Reaction of maltose | -0.001 |
| (d) 1,5-AG + maltose | 0.131 |

As shown in Table 11, the value of (c) closely agreed with that of (a), indicating that maltose contained in solution (c) was eliminated by the method of the invention. Further, the value of (b) closely agreed with that of (d). The utility of the method according to the invention was thus proved.

Example 22

Samples respectively containing 25 μg/ml 1,5-AG plus maltose at the concentrations indicated in Table 12 were prepared. To 0.075 ml of each of the samples was added 2.25 ml of reagent 1 prepared in Example 19, followed by incubation at 37°C for 5 minutes. After 0.75 ml of reagent 2 prepared in Example 19 was added to each mixture, the reaction was carried out for 5 minutes. The absorbance was measured at 438 nm, and 1,5-AG was determined using the calibration curve obtained in Example 20. The results are shown in Table 12.

Table 12

| Maltose (mg/dl) | 1,5-AG value (μg/ml) | Maltose effect (%) |
|---|---|---|
| 0 | 25.4 | 0 |
| 80 | 25.2 | 0.8 |
| 160 | 25.3 | 0.4 |
| 240 | 25.5 | 0.4 |
| 320 | 25.4 | 0 |
| 400 | 25.2 | 0.8 |

It was demonstrated that the concentration of 1,5-AG in a sample containing maltose even at a high concentration (400 mg/dl) could be accurately measured by using the reagents prepared in Example 19. The maximal effect rate of maltose was 0.8%, and the obtained 1,5-AG values were clinically satisfactory.

Example 23

Reagents for the determination of 1,5-AG having the following compositions were prepared.

| Reagent 1 | |
|---|---|
| ADA buffer (pH 7.0) | 20 mmol/l |
| Magnesium acetate | 8 mmol/l |
| NADP (Oriental Yeast Co., Ltd.) | 10 mmol/l |
| ADP (Oriental Yeast Co., Ltd.) | 0.5 mmol/l |
| DIP (derived from Clostridium sp., Toyobo Co., Ltd.) | 3 KU/l |
| MER (derived from Lactobacillus brevis, Kikkoman Corporation) | 20 KU/l |
| MP (derived from recombinant E. coli, Kikkoman Corporation) | 12 KU/l |
| Mutarotase (derived from pig kidney, Oriental Yeast Co., Ltd.) | 10 KU/l |
| GOD (derived from Aspergillus sp., Toyobo Co., Ltd.) | 300 KU/l |
| Catalase (derived from bovine liver, Sigma Chemical Co.) | 100 KU/l |

| Reagent 2 | |
|---|---|
| TAPS buffer (pH 8.5) | 200 mmol/l |
| WST-1 (Dojindo Laboratories) | 0.6 mmol/l |
| ADP-HK (derived from Thermococcus litoralis, Asahi Kasei Corporation) | 40 KU/l |
| AG-6PDH [derived from E. coli DF1 (ATCC 33849), Asahi Kasei Corporation] | 50 KU/l |

Example 24

1,5-AG was dissolved in purified water to prepare standard solutions respectively having the 1,5-AG concentrations of 0, 10, 20, 30, 40 and 50 μg/ml. To 0.075 ml of each of the solutions was added 2.25 ml of reagent 1 prepared in Example 23, followed by incubation at 37°C for 5 minutes. After 0.75 ml of reagent 2 prepared in Example 23 was added to each mixture, the reaction was carried out for 5 minutes. The absorbance was measured at 438 nm and a calibration curve was obtained. The result is shown in Fig. 6.

The calibration curve obtained by using the reagents prepared in Example 23 showed almost a linear relationship between 1,5-AG concentration and absorbance.

Example 25

Reagent 1 prepared in Example 23 was poured into test tubes in 2.25 ml portions. To the test tubes were respectively added 0.075 ml each of (a) purified water (blank), (b) a solution containing 25 µg/ml 1,5-AG, (c) a solution containing 400 mg/dl maltose, and (d) a solution containing 25 µg/ml 1,5-AG and 400 mg/dl maltose, followed by incubation at 37°C for 5 minutes. After 0.75 ml of reagent 2 prepared in Example 23 was added to each mixture, the reaction was carried out for 5 minutes and the absorbance was measured at 438 nm. The results are shown in Table 13.

Table 13

| Sample | Absorbance (Abs) |
|---|---|
| (a) Purified water (blank) | 0.028 |
| (b) 25 µg/ml 1,5-AG | 0.112 |
| (c) 400 mg/dl Maltose | 0.028 |
| (c)-(a) Reaction of maltose | 0.000 |
| (d) 1,5-AG + maltose | 0.112 |

As shown in Table 13, the value of (c) agreed with that of (a), indicating that maltose contained in solution (c) was eliminated by the method of the invention. Further, the value of (b) agreed with that of (d). The utility of the method according to the invention was thus proved.

Example 26

Samples respectively containing 25 µg/ml 1,5-AG plus maltose at the concentrations indicated in Table 14 were prepared. To 0.075 ml of each of the samples was added 2.25 ml of reagent 1 prepared in Example 23, followed by incubation at 37°C for 5 minutes. After 0.75 ml of reagent 2 prepared in Example 23 was added to each mixture, the reaction was carried out for 5 minutes. The absorbance was measured at 438 nm, and 1,5-AG was determined using the calibration curve obtained in Example 24. The results are shown in Table 14.

Table 14

| Maltose (mg/dl) | 1,5-AG value (µg/ml) | Maltose effect (%) |
|---|---|---|
| 0 | 25.5 | 0 |
| 80 | 25.1 | 1.6 |
| 160 | 25.3 | 0.8 |
| 240 | 25.8 | 1.2 |
| 320 | 25.6 | 0.4 |
| 400 | 25.6 | 0.4 |

It was demonstrated that the concentration of 1,5-AG in a sample containing maltose even at a high concentration (400 mg/dl) could be accurately measured by using the reagents prepared in Example 23. The maximal effect rate of maltose was 1.6%, and the obtained 1,5-AG values were clinically satisfactory.

Example 27

Reagents for the determination of 1,5-AG having the following compositions were prepared.

| Reagent 1 | |
|---|---|
| ADA buffer (pH 7.0) | 20 mmol/l |
| Magnesium acetate | 8 mmol/l |
| NADP (Oriental Yeast Co., Ltd.) | 10 mmol/l |
| ATP (Kyowa Hakko Kogyo Co., Ltd.) | 10 mmol/l |
| DIP (derived from Clostridium sp., Toyobo Co., Ltd.) | 3 KU/l |
| α-GH (derived from Bacillus stearothermophilus, Toyobo Co., Ltd.) | 5 KU/l |
| Mutarotase (derived from pig kidney, Oriental Yeast Co., Ltd.) | 10 KU/l |
| GOD (derived from Aspergillus sp., Toyobo Co., Ltd.) | 300 KU/l |
| Catalase (derived from bovine liver, Sigma Chemical Co., Ltd.) | 100 KU/l |

| Reagent 2 | |
|---|---|
| TAPS buffer (pH 8.5) | 200 mmol/l |
| WST-1 (Dojindo Laboratories) | 0.6 mmol/l |
| HK (derived from Thermococcus litoralis, Asahi Kasei Corporation) | 400 KU/l |
| AG-6PDH [derived from E. coli DF1 (ATCC 33849), Asahi Kasei Corporation] | 50 KU/l |

Example 28

1,5-AG was dissolved in purified water to prepare standard solutions respectively having the 1,5-AG concentrations of 0, 50, 100, 150, 200 and 250 µg/ml. To 0.075 ml of each of the solutions was added 2.25 ml of reagent 1 prepared in Example 27, followed by incubation at 37°C for 5 minutes. After 0.75 ml of reagent 2 prepared in Example 27 was added to each mixture, the reaction was carried out for 5 minutes. The absorbance was measured at 438 nm and a calibration curve was obtained. The result is shown in Fig. 7.

The calibration curve obtained by using the reagents prepared in Example 27 showed almost a linear relationship between 1,5-AG concentration and absorbance.

Example 29

Reagent 1 prepared in Example 27 was poured into test tubes in 2.25 ml portions. To the test tubes were respectively added 0.075 ml each of (a) purified water (blank), (b) a solution containing 250 µg/ml 1,5-AG, (c) a solution containing 400 mg/dl maltose, and (d) a solution containing 250 µg/ml 1,5-AG and 400 mg/dl maltose, followed by incubation at 37°C for 5 minutes. After 0.75 ml of reagent 2 prepared in Example 27 was added to each mixture, the reaction was carried out for 5 minutes and the absorbance was measured at 438 nm. The results are shown in Table 15.

Table 15

| Sample | Absorbance (Abs) |
|---|---|
| (a) Purified water (blank) | 0.305 |
| (b) 250 µg/ml 1,5-AG | 0.462 |
| (c) 400 mg/dl Maltose | 0.306 |
| (c)-(a) Reaction of maltose | 0.001 |
| (d) 1,5-AG + maltose | 0.463 |

As shown in Table 15, the value of (c) closely agreed with that of (a), indicating that maltose contained in solution (c) was eliminated by the method of the invention. Further, the value of (b) closely agreed with that of (d). The utility of the method according to the invention was thus proved.

Example 30

Samples respectively containing 250 μg/ml 1,5-AG plus maltose at the concentrations indicated in Table 16 were prepared. To 0.075 ml of each of the samples was added 2.25 ml of reagent 1 prepared in Example 27, followed by incubation at 37°C for 5 minutes. After 0.75 ml of reagent 2 prepared in Example 27 was added to each mixture, the reaction was carried out for 5 minutes. The absorbance was measured at 438 nm, and 1,5-AG was determined using the calibration curve obtained in Example 28. The results are shown in Table 16.

Table 16

| Maltose (mg/dl) | 1,5-AG value (μg/ml) | Maltose effect (%) |
| --- | --- | --- |
| 0 | 250.9 | 0 |
| 80 | 251.2 | 0.1 |
| 160 | 252.1 | 0.5 |
| 240 | 252.3 | 0.6 |
| 320 | 252.1 | 0.5 |
| 400 | 252.5 | 0.6 |

It was demonstrated that the concentration of 1,5-AG in a sample containing maltose even at a high concentration (400 mg/dl) could be accurately measured by using the reagents prepared in Example 27. The maximal effect rate of maltose was 0.6%, and the obtained 1,5-AG values were clinically satisfactory.

Example 31

Reagents for the determination of 1,5-AG having the following compositions were prepared.

| Reagent 1 | |
| --- | --- |
| ADA buffer (pH 7.0) | 20 mmol/l |
| Magnesium acetate | 8 mmol/l |
| NADP (Oriental Yeast Co., Ltd.) | 10 mmol/l |
| ATP (Kyowa Hakko Kogyo Co., Ltd.) | 10 mmol/l |
| DIP (derived from Clostridium sp., Toyobo Co., Ltd.) | 3 KU/l |
| MER (derived from Lactobacillus brevis, Kikkoman Corporation) | 20 KU/l |
| MP (derived from recombinant E. coli, Kikkoman Corporation) | 12 KU/l |
| Mutarotase (derived from pig kidney, Oriental Yeast Co., Ltd.) | 10 KU/l |
| GOD (derived from Aspergillus sp., Toyobo Co., Ltd.) | 300 KU/l |
| Catalase (derived from bovine liver, Sigma Chemical Co.) | 100 KU/l |

| Reagent 2 | |
| --- | --- |
| TAPS buffer (pH 8.5) | 200 mmol/l |
| WST-1 (Dojindo Laboratories) | 0.6 mmol/l |

(continued)

| Reagent 2 | |
|---|---|
| HK (derived from Thermococcus litoralis, Asahi Kasei Corporation) | 400 KU/l |
| AG-6PDH [derived from E. coli DF1 (ATCC 33849), Asahi Kasei Corporation] | 50 KU/l |

Example 32

1,5-AG was dissolved in purified water to prepare standard solutions respectively having the 1,5-AG concentrations of 0, 50, 100, 150, 200 and 250 µg/ml. To 0.075 ml of each of the solutions was added 2.25 ml of reagent 1 prepared in Example 31, followed by incubation at 37°C for 5 minutes. After 0.75 ml of reagent 2 prepared in Example 31 was added to each mixture, the reaction was carried out for 5 minutes. The absorbance was measured at 438 nm and a calibration curve was obtained. The result is shown in Fig. 8.

The calibration curve obtained by using the reagents prepared in Example 31 showed almost a linear relationship between 1,5-AG concentration and absorbance.

Example 33

Reagent 1 prepared in Example 31 was poured into test tubes in 2.25 ml portions. To the test tubes were respectively added 0.075 ml each of (a) purified water (blank), (b) a solution containing 250 µg/ml 1,5-AG, (c) a solution containing 400 mg/dl maltose, and (d) a solution containing 250 µg/ml 1,5-AG and 400 mg/dl maltose, followed by incubation at 37°C for 5 minutes. After 0.75 ml of reagent 2 prepared in Example 31 was added to each mixture, the reaction was carried out for 5 minutes and the absorbance was measured at 438 nm. The results are shown in Table 17.

Table 17

| Sample | Absorbance (Abs) |
|---|---|
| (a) Purified water (blank) | 0.312 |
| (b) 250 µg/ml 1,5-AG | 0.420 |
| (c) 400 mg/dl Maltose | 0.312 |
| (c)-(a) Reaction of maltose | 0.000 |
| (d) 1,5-AG + maltose | 0.420 |

As shown in Table 17, the value of (c) agreed with that of (a), indicating that maltose contained in solution (c) was eliminated by the method of the invention. Further, the value of (b) agreed with that of (d). The utility of the method according to the invention was thus proved.

Example 34

Samples respectively containing 250 µg/ml 1,5-AG plus maltose at the concentrations indicated in Table 18 were prepared. To 0.075 ml of each of the samples was added 2.25 ml of reagent 1 prepared in Example 31, followed by incubation at 37°C for 5 minutes. After 0.75 ml of reagent 2 prepared in Example 31 was added to each mixture, the reaction was carried out for 5 minutes. The absorbance was measured at 438 nm, and 1,5-AG was determined using the calibration curve obtained in Example 32. The results are shown in Table 18.

Table 18

| Maltose (mg/dl) | 1,5-AG value (µg/ml) | Maltose effect (%) |
|---|---|---|
| 0 | 251.8 | 0 |
| 80 | 251.4 | 0.2 |
| 160 | 251.9 | 0 |
| 240 | 252.4 | 0.2 |

24

Table 18   (continued)

| Maltose (mg/dl) | 1,5-AG value (µg/ml) | Maltose effect (%) |
|---|---|---|
| 320 | 252.6 | 0.3 |
| 400 | 252.2 | 0.2 |

It was demonstrated that the concentration of 1,5-AG in a sample containing maltose even at a high concentration (400 mg/dl) could be accurately measured by using the reagents prepared in Example 31. The maximal effect rate of maltose was 0.3%, and the obtained 1,5-AG values were clinically satisfactory.

## Claims

1. A method for determining 1,5-anhydroglucitol (hereinafter referred to as 1,5-AG) in a sample containing 1,5-AG, maltose and glucose, which comprises:

   converting maltose in the sample into glucose using an enzyme system capable of converting maltose into glucose [hereinafter referred to as enzyme system (A)];
   converting glucose into a compound which is not phosphorylated by 1,5-anhydroglucitol 6-phosphorylating enzyme system (hereinafter referred to as AG-6P-ES) or dehydrogenated by the action of 1,5-anhydroglucitol-6-phosphate dehydrogenase (hereinafter referred to as AG-6PDH), using an enzyme system capable of converting glucose into said compound [hereinafter referred to as enzyme system (B)];
   converting 1,5-AG into 1,5-anhydroglucitol-6-phosphate (hereinafter referred to as 1,5-AG-6P) using the AG-6P-ES; dehydrogenating the formed 1,5-AG-6P with AG-6PDH in the presence of an oxidized coenzyme; and determining the component formed or reduced by the dehydrogenation reaction.

2. The method according to claim 1, wherein the AG-6P-ES is selected from the group consisting of (a) hexokinase and NTP, (b) glucokinase and NTP, and (c) NDP-dependent hexokinase and NDP.

3. The method according to claim 1, wherein enzyme system (A) is selected from the group consisting of (a) $\alpha$ -glucosidase, (b) maltose phosphorylase and inorganic phosphorus, and (c) maltose phosphorylase, inorganic phosphorus and maltose 1-epimerase.

4. The method according to claim 1, wherein enzyme system (B) is selected from the group consisting of (a) glucose 6-phosphorylating enzyme system (hereinafter referred to as G-6P-ES), phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

5. The method according to claim 1, wherein the AG-6P-ES is selected from the group consisting of (a) hexokinase and NTP, (b) glucokinase and NTP, and (c) NDP-dependent hexokinase and NDP; enzyme system (A) is selected from the group consisting of (a) $\alpha$-glucosidase, (b) maltose phosphorylase and inorganic phosphorus, and (c) maltose phosphorylase, inorganic phosphorus and maltose 1-epimerase; and enzyme system (B) is selected from the group consisting of (a) G-6P-ES, phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

6. The method according to claim 4 or 5, wherein the G-6P-ES is selected from the group consisting of (a) hexokinase and NTP, (b) glucokinase and NTP, and (c) NDP-dependent hexokinase and NDP.

7. The method according to claim 1, wherein the AG-6PDH is derived from a microorganism belonging to the genus Escherichia.

8. The method according to claim 1, wherein the formed component is a reduced coenzyme and the determination of the reduced coenzyme is carried out by measuring the absorbance of the reaction mixture obtained by dehydrogenation.

9. The method according to claim 1, wherein the formed component is a reduced coenzyme and the determination of the reduced coenzyme is carried out by subjecting the reduced coenzyme to reaction with a tetrazolium salt in the presence of an electron carrier and then measuring the absorbance of the reaction mixture colored by the formed formazan pigment.

**10.** A reagent for the determination of 1,5-AG, comprising:

a reagent for the conversion of maltose into glucose comprising enzyme system (A) capable of converting maltose into glucose;
a reagent for the elimination of glucose comprising enzyme system (B) capable of converting glucose into a compound which is not phosphorylated by AG-6P-ES or dehydrogenated by AG-6PDH;
a reagent for the conversion of 1,5-AG into 1,5-AG-6P comprising AG-6P-ES; and
a reagent for the dehydrogenation of 1,5-AG-6P comprising AG-6PDH and an oxidized coenzyme.

**11.** The reagent according to claim 10, wherein the AG-6P-ES is selected from the group consisting of (a) hexokinase and NTP, (b) glucokinase and NTP, and (c) NDP-dependent hexokinase and NDP.

**12.** The reagent according to claim 10, wherein enzyme system (A) is selected from the group consisting of (a) $\alpha$-glucosidase, (b) maltose phosphorylase and inorganic phosphorus, and (c) maltose phosphorylase, inorganic phosphorus and maltose 1-epimerase.

**13.** The reagent according to claim 10, wherein enzyme system (B) is selected from the group consisting of (a) G-6P-ES, phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

**14.** The reagent according to claim 10, wherein the AG-6P-ES is selected from the group consisting of (a) hexokinase and NTP, (b) glucokinase and NTP, and (c) NDP-dependent hexokinase and NDP; enzyme system (A) is selected from the group consisting of (a) $\alpha$-glucosidase, (b) maltose phosphorylase and inorganic phosphorus, and (c) maltose phosphorylase, inoganic phosphorus and maltose 1-epimerase; and enzyme system (B) is selected from the group consisting of (a) G-6P-ES, phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

**15.** The reagent according to claim 13 or 14,
wherein the G-6P-ES is selected from the group consisting of (a) hexokinase and NTP, (b) glucokinase and NTP, and (c) NDP-dependent hexokinase and NDP.

**16.** The reagent according to claim 10, wherein the AG-6PDH is derived from a microorganism belonging to the genus Escherichia.

**17.** A reagent kit for the determination of 1,5-AG, comprising:

a first container which contains a reagent for the conversion of maltose into glucose comprising enzyme system (A) capable of converting maltose into glucose, a reagent for the elimination of glucose comprising enzyme system (B) capable of converting glucose into a compound which is not phosphorylated by AG-6P-ES or dehydrogenated by AG-6PDH, and a reagent for the conversion of 1,5-AG into 1,5-AG-6P comprising AG-6P-ES; and
a second container which contains a reagent for the dehydrogenation of 1,5-AG-6P comprising AG-6PDH and an oxidized coenzyme.

**18.** A reagent kit for the determination of 1,5-AG, comprising:

a first container which contains a reagent for the conversion of maltose into glucose comprising enzyme system (A) capable of converting maltose into glucose, and a reagent for the elimination of glucose comprising enzyme system (B) capable of converting glucose into a compound which is not phosphorylated by AG-6P-ES or dehydrogenated by AG-6PDH; and
a second container which contains a reagent for the conversion of 1,5-AG into 1,5-AG-6P comprising AG-6P-ES, and a reagent for the dehydrogenation of 1,5-AG-6P comprising AG-6PDH and an oxidized coenzyme.

**19.** A reagent kit for the determination of 1,5-AG, comprising:

a first container which contains a reagent for the conversion of maltose into glucose comprising enzyme system (A) capable of converting maltose into glucose, a reagent for the elimination of glucose comprising enzyme system (B) capable of converting glucose into a compound which is not phosphorylated by AG-6P-ES or dehydrogenated by AG-6PDH, a reagent for the conversion of 1,5-AG into 1,5-AG-6P comprising AG-6P-ES, and an oxidized coenzyme; and
a second container which contains AG-6PDH.

**20.** A reagent kit for the determination of 1,5-AG, comprising:

a first container which contains a reagent for the conversion of maltose into glucose comprising enzyme system (A) capable of converting maltose into glucose, a reagent for the elimination of glucose comprising enzyme system (B) capable of converting glucose into a compound which is not phosphorylated by AG-6P-ES or dehydrogenated by AG-6PDH, and an oxidized coenzyme; and
a second container which contains a reagent for the conversion of 1,5-AG into 1,5-AG-6P comprising AG-6P-ES, and AG-6PDH.

**21.** A reagent kit for the determination of 1,5-AG, comprising:

a first container which contains a reagent for the conversion of maltose into glucose comprising enzyme system (A) capable of converting maltose into glucose, a reagent for the elimination of glucose and the conversion of 1,5-AG into 1,5-AG-6P comprising a member selected from the group consisting of (a) hexokinase and NTP, (b) glucokinase and NTP, and (c) NDP-dependent hexokinase and NDP, and phosphohexose isomerase, phosphofructokinase, NTP and an oxidized coenzyme; and
a second container which contains AG-6PDH.

**22.** A reagent kit for the determination of 1,5-AG, comprising:

a first container which contains a reagent for the conversion of maltose into glucose comprising enzyme system (A) capable of converting maltose into glucose, a reagent for the elimination of glucose comprising enzyme system (B) selected from the group consisting of (b) glucose oxidase, (c) glucose oxidase and mutarotase, (d) glucose oxidase and catalase, and (e) glucose oxidase, mutarotase and catalase, and NTP or NDP and an oxidized coenzyme; and
a second container which contains an enzyme capable of converting 1,5-AG into 1,5-AG-6P, and AG-6PDH.

**23.** The reagent kit according to any of claims 17 to 20, wherein the AG-6P-ES is selected from the group consisting of (a) hexokinase and NTP, (b) glucokinase and NTP, and (c) NDP-dependent hexokinase and NDP.

**24.** The reagent kit according to any of claims 17 to 22, wherein enzyme system (A) is selected from the group consisting of (a) $\alpha$-glucosidase, (b) maltose phosphorylase and inorganic phosphorus, and (c) maltose phosphorylase, inorganic phosphorus and maltose 1-epimerase.

**25.** The reagent kit according to any of claims 17 to 20, wherein enzyme system (B) is selected from the group consisting of (a) G-6P-ES, phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

**26.** The reagent kit according to any of claims 17 to 20, wherein the AG-6P-ES is selected from the group consisting of (a) hexokinase and NTP, (b) glucokinase and NTP, and (c) NDP-dependent hexokinase and NDP; enzyme system (A) is selected from the group consisting of (a) $\alpha$ -glucosidase, (b) maltose phosphorylase and inorganic phosphorus, and (c) maltose phosphorylase, inoganic phosphorus and maltose 1-epimerase; and enzyme system (B) is selected from the group consisting of (a) G-6P-ES, phosphohexose isomerase, 6-phosphofructokinase and NTP, (b) glucose oxidase, (c) glucose oxidase and mutarotase, (d) glucose oxidase and catalase, and (e) glucose oxidase, mutarotase and catalase.

**27.** The reagent kit according to claim 25 or 26, wherein the G-6P-ES is selected from the group consisting of (a) hexokinase and NTP, (b) glucokinase and NTP, and (c) NDP-dependent hexokinase and NDP.

**28.** The reagent kit according to any of claims 17 to 22, wherein the AG-6PDH is derived from a microorganism belonging to the genus <u>Escherichia.</u>

**29.** The reagent kit according to any of claims 17 to 22, wherein the first container further contains an electron carrier and the second container further contains a tetrazolium salt.

**30.** A method for determining 1,5-AG in a sample containing 1,5-AG, maltose and glucose, which comprises:

converting maltose in the sample into glucose with α-glucosidase in an aqueous medium;
converting glucose in the sample and glucose formed in the medium into fructose-1,6-diphosphate with ADP-dependent hexokinase, phosphohexose isomerase and 6-phosphofructokinase in the presence of NDP, NTP and ADP;
converting 1,5-AG in the sample into 1,5-AG-6P with ADP-dependent hexokinase in the presence of ADP;
dehydrogenating the formed 1,5-AG-6P with AG-6PDH in the presence of an oxidized coenzyme; and
determining the amount of the reduced coenzyme formed in the reaction mixture.

**31.** A method for eliminating maltose and glucose in a sample containing maltose and glucose, in a method for the determination of 1,5-AG using an enzyme, which comprises converting maltose into glucose using enzyme system (A) capable of converting maltose into glucose and converting glucose into fructose 1,6-diphosphate using G-6P-ES, phosphohexose isomerase, 6-phosphofructokinase and NTP.

**32.** The method according to claim 31, wherein said G-6P-ES is selected from the group consisting of (a) hexokinase and NTP, (b) glucokinase and NTP, and (c) NDP-dependent hexokinase and NDP.

**33.** A reagent for the elimination of maltose and glucose in a method for the determination of 1,5-AG using an enzyme, which comprises a reagent for the conversion of maltose into glucose comprising enzyme system (A) capable of converting maltose into glucose and a reagent for the conversion of glucose into fructose 1,6-diphosphate comprising G-6P-ES, phosphohexose isomerase, 6-phosphofructokinase and NTP.

**34.** The reagent according to claim 33, wherein said G-6P-ES is selected from the group consisting of (a) hexokinase and NTP, (b) glucokinase and NTP, and (c) NDP-dependent hexokinase and NDP.

Fig. 1

Fig. 2

Fig. 3

Fig. 4

Fig. 5

Fig. 6

Fig. 7

Fig. 8